# EUROPEAN PATENT APPLICATION

(11) **EP 1 752 132 A2**
(43) Date of publication of application: **14.02.2007**
(21) Application number: 06014265.0
(22) Date of filing: 10.07.2006
(51) Int. Cl.: A61K 8/67, A61Q 19/00

(54) **Skin cosmetic compositions**

(30) Priority: 08.08.2005 CN 200510089755
(71) Applicant: Lotus Pharmaceutical Co., Ltd., Taipei 106 (TW)
(72) Inventor: Lin, Tong Ho, Taipei (TW)
(74) Representative: Schoppe, Fritz

(57) **Abstract**

A composition for use with the skin. The composition includes a vitamin C in an amount from about 1% to 45% by weight, a vitamin B complex in an amount from about 1% to 5% by weight, a carotene in an amount from about 0.1% to 3% by weight, and a vitamin E in an amount from about 2% to 90% by weight. The composition is for topical application and is useful in skin-care. The composition is used for keeping the moisture of the skin and treating acnes, comedos, and zits. The composition is also an antioxidant.

## Description

### FIELD OF THE INVENTION

The present invention relates to a composition for topical usage. The composition can be used for nourishing a skin, keeping the moisture of a skin and treating acnes, comedos and zits. The composition has an antioxidation property and is free of vitamin A acid.

### BACKGROUND OF THE INVENTION

The structure of a human skin includes the epidermis, dermis, subcutaneous fatty tissues, sebaceous glands, sweat glands, hairs and nails. The thick layer under the epidermis is named as the dermis. The hairs are surrounded by the hair follicles, and there are sebaceous glands nearby. The sebaceous glands can secrete sebum, and the sebum will permeate through the surface of the skin via the hairs and hair follicles. Then the sebum becomes fatty films, which attach to the surface of the skin and protect the skin. The type of a skin is sorted by the quantity of sebaceous glands, and the types are usually classified as oily skins, dry-type skins and mixed-type skins.

An acne is a chronic inflammation in hair follicles and sebaceous glands, which is also commonly known as a comedo or a zit. It is usually found on the forehead, the surroundings of the nosewing, cheeks, and any part of a human body with hair follicles, such as the back, the chest and thighs. Depending on the symptom, an acne can be classified as an acne type, an inflammatory and swollen type, and a cyst type. When gonads of teenagers become mature, the quantity of androgenic hormones in testes and ovaries will increase, and the androgenic hormones will stimulate sebaceous glands of a human skin. The sebaceous glands will become hypertrophy and secrete an abundance of sebum, and the sebum will collect around sebaceous glands and hair follicles. Then the skin will be inflamed due to the infection of bacteria. At first, a white-head acne or a black-head acne is formed, and subsequently it becomes a papule, a pustule, a node or a cyst after being infected by bacteria which is known as a zit. When the secretion of androgenic hormones is too much, the T area of the face or the skin under the eyelids may become red, tickled, fevered, hot and peeled. Sometimes even a blood capillary becomes visible. The symptom is called seborrheic dermatitis.

The preparations for external usage are expected to clean a skin, kill bacteria on a skin, prevent water evaporating from a skin and improve the moisture of a skin. For example, a cream containing sodium chloride for softening a skin is disclosed in US patent No. 3,574,854. A composition with mineral salts for cleaning a skin is disclosed in German patent publication No. 3,327,840. A glucose mixture for lubricating a skin is disclosed in US patent No. 3,859,436, and a glucose solution for shaving is disclosed in US patent No. 3,777,597.

It is known that the sodium chloride is the main composition to maintain the osmotic pressure of body fluids, especially the 0.9% sodium chloride solution is called the physiological saline. However, the sodium chloride solution with high concentration can not be absorbed by a skin, and it will irritate the skin mucosa and thus result in dehydration. Therefore, the preparations containing sodium chloride is able to stimulate a skin or kill bacteria, but it is difficult to prevent water evaporating from the skin and keep the moisture of the skin.

The glucose, as a nutrient, may increase the level of glycogens, stimulate the hyperfunction of cells, and improve the metabolism of an organism. The glucose can also function as an antidote. However, it is difficult to externalize the above-described specific effects by applying the preparations containing glucose to skin, unless the glucose is supplemented by oral, intravenous injection or intramuscular injection.

It usually adds enhancers of transdermal absorption, such as dibutyl adipate, and the mixture of dibutyl adipate and isopropyl myristate, into pharmaceutical agents for topical usages. The isopropyl myristate is known as a permeation enhancer in topical usage formulation. However, the applicant does not find any synergistic effect of dibutyl adipate and isopropyl myristate from published documents.

There are many therapeutic agents and cosmetics for treating skin conditions, such as the hydrocortisone for treating the atopic dermatitis with titillate and erythema, the sulconazole nitrate for treating mycotic infections of a skin, the tretinoin for treating photo-aging, and the 5-fluorouracil for treating the psoriasis and the skin cancer. Usually, the permeation enhancers are applied for treating the dermatosis, such as dimethyl sulfoxide (DMSO), dimethyl formamide, methyldecyl sulfoxide (disclosed in US patent No. 3,527,864), dimethyl acetamide (disclosed in US patent No. 3,472,931) and N-alkyl-2-pyrrolidone (disclosed in US patent No. 3,696,516). However, the above-mentioned permeation enhancers have certain disadvantages. For example, the dimethyl sulfoxide has a peculiar smell and body odors, induce a burn and erythema on a skin, reduce the transparence of crystal and even cause tissue necrosis of an animal (Martindale. 1977. The extra pharmacopoeia, 27th ed., pp1461-1463). The dimethyl formamide and dimethyl acetamide can also induce a burn and an erythema on a skin.

In US patent No. 5,030,451, Trebosc *et al.* disclose a cosmetics composition containing modified derivatives of caffeine as active agents. The formulation have excellent and long-term lipolytic properties and have therefore been proven very effective in a slenderizing program and in the treatment of a cellulite. In US patent No. 5,215,759, a composition of methylsilanol theophyllinacetate alginate and methylsilanol mannuronate is used for anti-cellulite.

In US patent No. 5,051,449, it is disclosed that the cellulite can be slightly improved by topically applying a retinoid to the skin. It is observed an amelioration from moderate to obvious level of a thickened epidermis, an increased number of new blood vessels, and a pinching test.

In US patent application No. 20020099094, it is disclosed a topical aminolevulinic acid-photodynamic therapy for the treatment of acne vulgaris. In US patent application No.20020061855, a composition for treating acnes including water and glycol is disclosed. In US patent application No. 20010056071, it is disclosed a composition for treating acnes consisting of resveratrol (3,4',5-trihydroxy-trans-stilbene), melatonin, vitamin D, vitamin E, and vitamin A. In US patent No. 4,938,960, it is disclosed a composition for protecting a skin consisting of vitamin B complex, vitamin C, vitamin E and phospholipids.

It is disclosed that the combination of vitamin A, vitamin D and the derivatives, vitamins C, vitamins E, and coenzyme Q can be useful in improving the skin and curing acnes (Shapiro and Saliou. 2001. Nutrition, vol 17, p839-844). However, the actual formulation is not disclosed. All the above-mentioned vitamins are used for maintaining physiological activities of a human being. The need of vitamins is very small in the body, but the function of vitamins is very important. The vitamins can not be synthesized by a human body and need to be ingested from outside.

The vitamin A acid, also known as tretinoin or retinoids, is belonging to the derivatives of vitamin A according to its structure. The major function of vitamin A acid is as a drug for removing keratins. The vitamin A acid can remove keratins on the stratum corneum of a epidermis, thereby improving the occlusion of a pore. It can improve wrinkles of skin and blood circulation around the face, decrease the scar of pigments and prevent the keratinization of the skin. It can also promote the refreshing and sloughing off of epithelium cells, prevent the synthesis of keratins, and prevent the formation of blisters on the face. However, most vitamin A Acid-containing products may make the skin sensitive to the light, and will cause side-effects, such as drying, red swelling, itching and dermatitis.

The vitamin B complex is not only one vitamin but a combination of various vitamins, such as vitamin B₁ (thiamin), vitamin B₂ (riboflavin), Niacin, vitamin B₅ (pantothenic acid), vitamin B₆ (pyridoxal), folic acid, vitamin B₁₂ (cobalamin) and biotin. The major function of vitamin B complex is as a component of coenzymes, whose function is to facilitate the oxidation of glucose, fats and proteins to release energy. It also maintains the normal function of the nervous system. The vitamin B complex is necessary for the proliferation and regeneration of cells, the generation of erythrocytes and the synthesis of nucleoprotein and myelin. It can also activate the folic acid related enzymes so as to facilitate the generation of erythrocytes.

The major functions of vitamin C in the body are as follows: preventing the formation of peroxylipids, facilitating the formation of collagens, being coenzymes for many enzymes, retarding the aging of cells, improving the blood circulation and reducing melanins gradually. It is considered that the vitamin C contributes to the regeneration of the skin, prevents the generation of melanins and enhances the immunity.

The vitamin E is considered as an antioxidant which can inhibit the agglutination of blood platelets. The vitamin E can prevent the oxidation of the cell membrane of an erythrocyte so as to protect it from being destroyed for avoiding anemia. Moreover, the vitamin E can also maintain the integrity of the cell membrane and enhance the function of linoleic acid. The vitamin E can protect the structures and functions of muscles and nerve tissues, and thus increase the blood flow at terminal vessels and improve the situation of the blood flow. Although the vitamin E has the above-described activities, no commercial available products, which contain the vitamin E with the Wal Green, vitamin E-containing L'oreal Furtur E or Jasom Natural Cosmetics, have the functions of nourishing the skin, treating acnes, comedos and zits, or antioxidation.

It is described in US patent No. 5,834,445 that the carotene and the vitamin E can reach Langerhans cells of mucosas, and increase the topical immunity thereof. However, the carotene has a darker color and it is lipophilic. Therefore, there are technical problems in making creams, emulsions, lotions for use with the skin or cosmetics.

From the above descriptions, although there are some patents related to remedying acnes, decomposing fat or treating various skin symptoms, all compositions are different from the vitamin A acid-free composition for use with the skin according to the present invention.

In order to overcome the foresaid drawbacks in the prior arts, the present invention provides a composition for use with the skin.

### SUMMARY OF THE INVENTION

It is an aspect of the present invention to provide a topical composition for use with the skin. The composition is free of vitamin A acids and is useful in skin-care. The composition is used for keeping the moisture of the skin and treating acnes, comedos and zits. The composition is also an antioxidant.

According to the present invention, the composition for use with the skin includes a vitamin C in an amount from 1% to 45% by weight, a vitamin B complex in an amount from 1% to 5% by weight, a carotene in an amount from 0.1% to 3% by weight, a vitamin E in an amount from 2% to 90% by weight, a flavor in an amount from 0.1% to 2% by weight, a thickening agent in an amount from 1% to 5% by weight, a surfactant in an amount from about 1 % to 8% by weight and distilled water.

Preferably, the composition according to the present invention includes a vitamin C in an amount from 4% to 15% by weight, a vitamin B complex in an amount from 1% to 3% by weight, a carotene in an amount from 0.1% to 2% by weight and a vitamin E in an amount from 15% to 65% by weight.

The composition according to the present invention mainly includes a vitamin C, a vitamin B complex, a carotene, a vitamin E, a flavor, a thickening agent and a surfactant. The activity of the composition according to the present invention is distinguishable from that of single vitamin compound by oral. The composition according the present invention is applying to an affected site of acnes, comedos and zits, and it has the effects of antioxdation and treating acnes, comedos and zits. The composition according to the present invention is topically used with the face skin or the limb skin, and the composition has the functions of nourishing the skin and keeping the moisture of the skin.

The activity test demonstrates that the ratio of main components of the composition according to the present invention shows excellent efficacy. It should be noted that it can not be presumed from the conventional technology in the art, although the composition according to the present invention is consisted of a vitamin C, a vitamin B complex, a carotene, a vitamin E, a flavor, a thickening agent and a surfactant.

According to the composition of the present invention, various kinds of excipients, carriers or diluting agents could be added to formulate an ointment, a emulsion, a lotion or a patch which could apply to an affected site. An adhesive, such as a starch and a sodium carboxymethylcellulose, are added to those formulations by the conventional technique in the art. A buffer such as a phosphate is also added thereto so as to adjust the pH value to a proper range. The formulation is made by the conventional technique in the art. An enhancer for permeation or natural plants extracts such as a licorice root may be added thereto.

The main components of the composition according to the present invention are the essential vitamins, and the composition is free of vitamin A acid. Thus, there are no problems such as over-dosage and osteoporosis for long-term usage.

The above aspects and advantages of the present invention will become more readily apparent to those ordinarily skilled in the art after reviewing the following detailed descriptions and accompanying drawings, in which:

### BRIEF DESCRIPTION OF THE DRAWINGS

Fig. 1 is a diagram showing the effect of the composition according to a preferred embodiment of the present invention for keeping the moisture of the skin on the forehead.

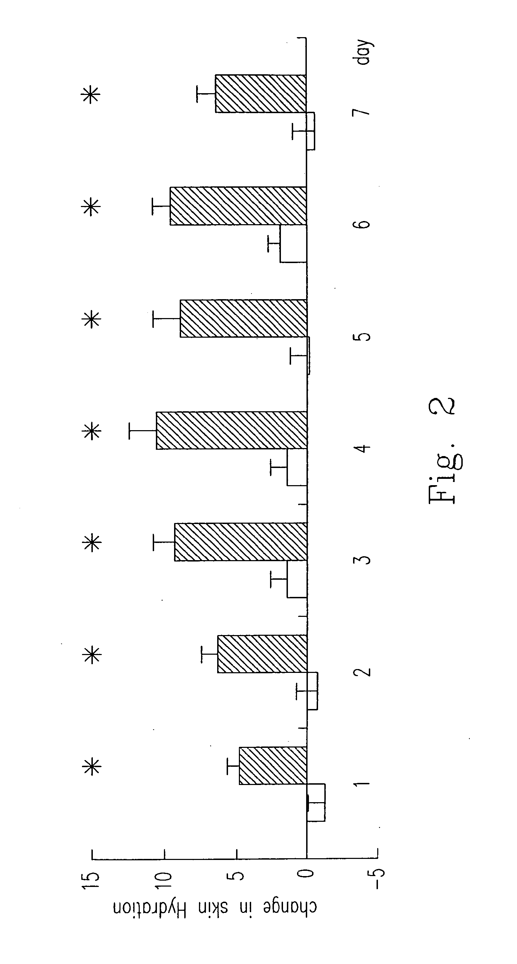

Fig. 2 is a diagram illustrating the effect of the composition according to a preferred embodiment of the present invention for keeping the moisture of the skin on cheeks.

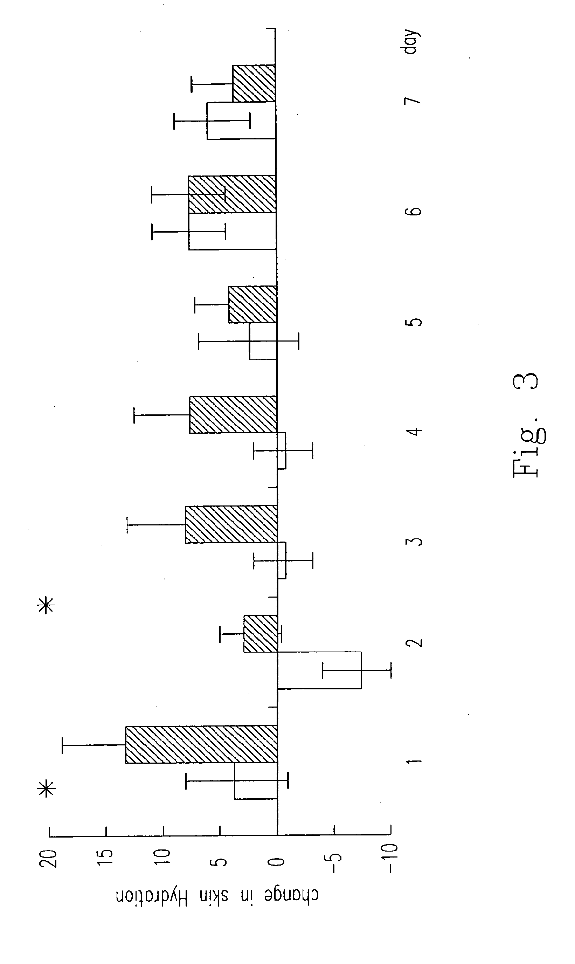

Fig. 3 is a diagram showing the effect of the composition according to another preferred embodiment of the present invention for keeping the moisture of the skin on the forehead.

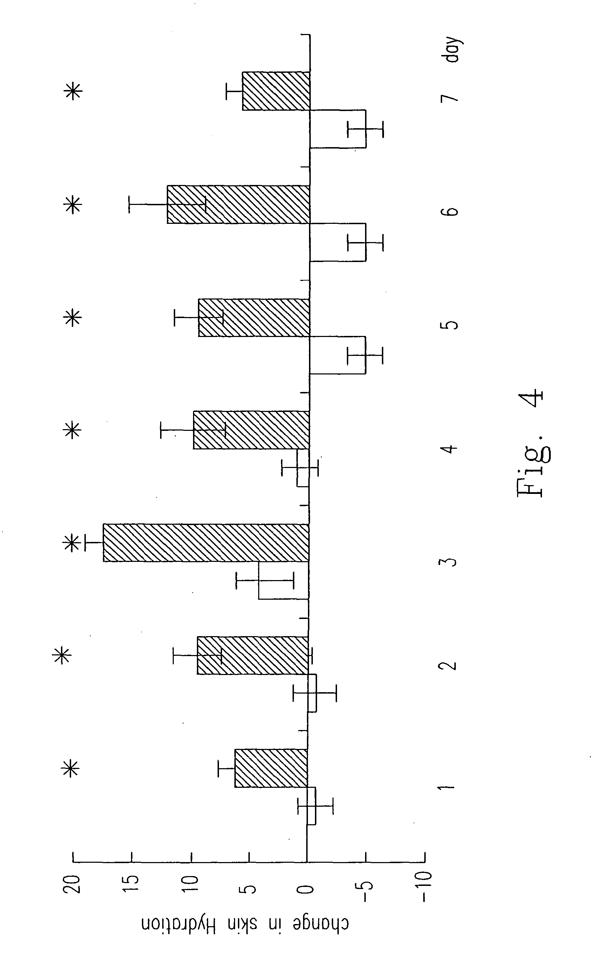

Fig. 4 is a diagram illustrating the effect of the composition according to another preferred embodiment of the present invention for keeping the moisture of the skin on cheeks.

### DETAILED DESCRIPTION OF THE PREFERRED EMBODIMENT

The invention will now be described more specifically with reference to the following embodiments. It is to be noted that the following descriptions of preferred embodiments of this invention are presented herein for the purposes of illustration and description only; it is not intended to be exhaustive or to be limited to the precise form disclosed.

The following are some formulations according to the present invention.

Example 1: the preparation of Formulation Lo-107

| Components | Ratio by weight |
|---|---|
| Vitamin E | 25 |
| Vitamin C | 20 |
| Vitamin B complex | 2 |
| Carotene | 0.1 |
| Flavor | 2 |
| Surfactant | 8.0 |
| Thickening agent | 1 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 2: the preparation of formulation Lo-108

| Components | Ratio by weight |
|---|---|
| Vitamin E | 65 |
| Vitamin C | 4 |
| Vitamin B complex | 1 |
| Carotene | 1 |
| Flavor | |
| Surfactant | 6.5 |
| Thickening agent | 4 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 3: the preparation of formulation Lo-109

| Components | Ratio by weight |
|---|---|
| Vitamin E | 40 |
| Vitamin C | 10 |
| Vitamin B complex | 3 |
| Carotene | 0.5 |
| Flavor | 1 |
| Surfactant | 8 |
| Thickening agent | 5 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 4: the preparation of formulation Lo-110

| Components | Ratio by weight |
|---|---|
| Vitamin E | 20 |
| Vitamin C | 20 |
| Vitamin B complex | 2 |
| Carotene | 2 |
| Flavor | 2 |
| Surfactant | 8 |
| Thickening agent | 1 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 5: the preparation of formulation Lo-122

| Components | Ratio by weight |
|---|---|
| Vitamin E | 85 |
| Vitamin C | 2 |
| Vitamin B complex | 1 |
| Carotene | 1 |
| Flavor | 0.1 |
| Surfactant | 3 |
| Thickening agent | 2 |
| Enhancer of permeation | 0.5 |

The components of a vitamin C and a vitamin B complex are dissolved in a small amount of distilled water. Meanwhile the amounts of a carotene, a vitamin E, a flavor, a surfactant, a thickening agent and an enhancer of permeation are mixed together. The two solutions are mixed together and then a proper amount of distilled water is added thereinto.

Example 6: the preparation of formulation Lo-130

| Components | Ratio by weight |
|---|---|
| Vitamin E | 40 |
| Vitamin C | 10 |
| Vitamin B complex | 3 |
| Carotene | 2 |
| Flavor | 2 |
| Surfactant | 10 |
| Thickening agent | 5 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 7: the preparation of formulation Lo-18

| Components | Ratio by weight |
|---|---|
| Vitamin E | 90 |
| Vitamin C | 1 |
| Vitamin B complex | 1 |
| Carotene | 1 |
| Flavor | 0.5 |
| Surfactant | 3 |
| Thickening agent | 3 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 8: the preparation of formulation Lo-27

| Components | Ratio by weight |
|---|---|
| Vitamin E | 30 |
| Vitamin C | 45 |
| Vitamin B complex | 1 |
| Carotene | 2 |
| Flavor | 2 |
| Surfactant | 8 |
| Thickening agent | 1 |
| Enhancer of permeation | 0.1 |

The components of a vitamin C and a vitamin B complex are dissolved in a small amount of distilled water. Meanwhile the amounts of a carotene, a vitamin E, a flavor, a surfactant, a thickening agent and an enhancer of permeation are mixed together. The two solutions are mixed together and then a proper amount of distilled water is added thereinto.

Example 9: the preparation of formulation Lo-22

| Components | Ratio by weight |
|---|---|
| Vitamin E | 80 |
| Vitamin C | 8 |
| Vitamin B complex | 4 |
| Carotene | 3 |
| Flavor | 0.1 |
| Surfactant | 3 |
| Thickening agent | 2 |

The components of a vitamin C and a vitamin B complex are dissolved in a small amount of distilled water. Meanwhile the amounts of a carotene, a vitamin E, a flavor, a surfactant and a thickening agent are mixed together. The two solutions are mixed together and then a proper amount of distilled water is added thereinto.

Example 10: the preparation of formulation Lo-39

| Components | Ratio by weight |
|---|---|
| Vitamin E | 30 |
| Vitamin C | 10 |
| Vitamin B complex | 5 |
| Carotene | 2 |
| Flavor | 2 |
| Surfactant | 10 |
| Thickening agent | 5 |
| Enhancer of permeation | 1.5 |

The components of a vitamin C and a vitamin B complex are dissolved in a small amount of distilled water. Meanwhile the amounts of a carotene, a vitamin E, a flavor, a surfactant, a thickening agent and an enhancer for permeation are mixed together. The two solutions are mixed together and then a proper amount of distilled water is added thereinto.

The following are the experiments on the activity of the compositions according to the present invention.

Experiment 1: the evaluation of the therapeutic efficacy of Lo-108 for the patients suffering from acnes

An open clinical efficacy evaluation method is used. Sixty patients suffering from acnes are selected during Feb. to Aug. in 2001 from the outpatients, including 30 males and 30 females. Their ages are between 17-42 year-old and the average age is 25 year-old. The Lo-108 is used over six months by topical application, and it is applied to the face for 3 to 8 hours (i.e. overnight) everyday. The patients are recorded the number and property changes of acnes every two weeks. The applying methods are as follows:

1. The affected part is washed and cleared with the washer milk (soap), and then the composition is applied thereto after it dries.

2. The dosage for application is about 2 ml, and then the affected part is covered with a bandage.

3. The composition is washed off after having been administrated for 3 hours, and no emulsion (cream) is allowed after washing.

4. If there is a pustule, it should be squeezed before the composition is applied.

If the patient has a pustule, an antibiotics such as a tetracycline or a vibramycin is needed to be administrated orally for a week. Other therapies for acnes, such as vitamin A acids for oral administration or topical use, hormone therapy, are prohibited in the rest of the time. The result is evaluated by the doctor and patients themselves according to the therapy ratio and degree which is scaled by the number of acnes, papules and pustules, and the degree of the red swelling of the skin.

**Table 1. The results of the evaluation of the therapeutic efficacy of Lo-108**

| | Before applied (number) | After applied over 8 weeks(number) |
|---|---|---|
| mean no. of comedos | 43.5 | 20.1(p<0.01) |
| mean no. of papules | 21.0 | 2.1(p<0.001) |
| mean no. of pustules | 8.9 | 0(p<0.0001) |
| mean no. of cysts | 0.8 | 0 |

The results are shown in Table 1. After applied for over 8 weeks, the number of black-head or white-head acnes decreases significantly and the average number changes from 43.5 to 20.1 (p<0.01). Besides, the number of red papules with contracted pore-opening reduces significantly and the average number changes from 21.0 to 2.1 (p<0.001). The pustules and the red swelling of cysts disappear and the average number changes from 8.9 to 0, or from 0.8 to 0, and only some erythemas or dented scars remain. The reduction of the red swelling occurs in the first four weeks, and most examinees can feel the significant improvement and would like to continue using the composition. During the evaluation, it is found that the Lo-108 not only eliminates the inflammation, reduces the temperature and weakens the swelling, but also inhibits the hyperplasia tendency of hyperkeratosis of the skin, and then makes the skin more smooth. The improvement of dented scars only occurs in the patients of the amelioration of swelling, and this is probably an impression due to the detumescence.

The topical medicines for de-keratinization or antibiotics used in the art usually have many side effects, such as a dryness, a peel, a smart and even a red swelling. These side effects do not appear after the Lo-108 is administrated because of its moisturizing function.

From the above-described results, it is concluded that the Lo-108 has therapeutic efficacy for acnes. The Lo-108 may also have the functions of de-keratinizing, reducing the excretion of sebum, killing bacteria, eliminating inflammation and increasing the water content in cuticles.

Experiment 2: the comparative evaluation of the inhibition of sebum excretion

Twenty healthy volunteers take part in this clinical test and their ages are from 18 to 55 year-old. One part of skins on examinees' foreheads is administrated with Lo-108, the other part serving as the control is administrated with nothing. The Lo-108 is administrated every night as follows: the Lo-108 is applied to skins with the dosage of 1-2 ml, and then it is washed off three hours later. The treatment continues for 4 weeks. The amount of sebum on both parts of the forehead skin is measured each week, and it is measured at a regular interval each time.

The test of sebum excretion amount is determined by measuring the amount of skin grease via the Sebometer 810 PC (Courage and Khazaka Ltd, Germany). Because the percentage difference of sebum is obtained by determining the sebum amount on both parts of the forehead skins, many variations can decrease to a minimum. The principle of determining sebum amount is as follows: a opaque plastic substance is provided, and its thickness and area are 0.1mm and 64mm² respectively. After the opaque plastic substance is depressed on the skin for 30 seconds, the transparency thereof increases because it absorbs the sebum. There is a linear correlation between the transparency and the amount of absorbed sebum. In other words, the amount of sebum is in direct proportion to the transparency, and the data obtained from the photometer can be converted into mg/cm² by a formula.

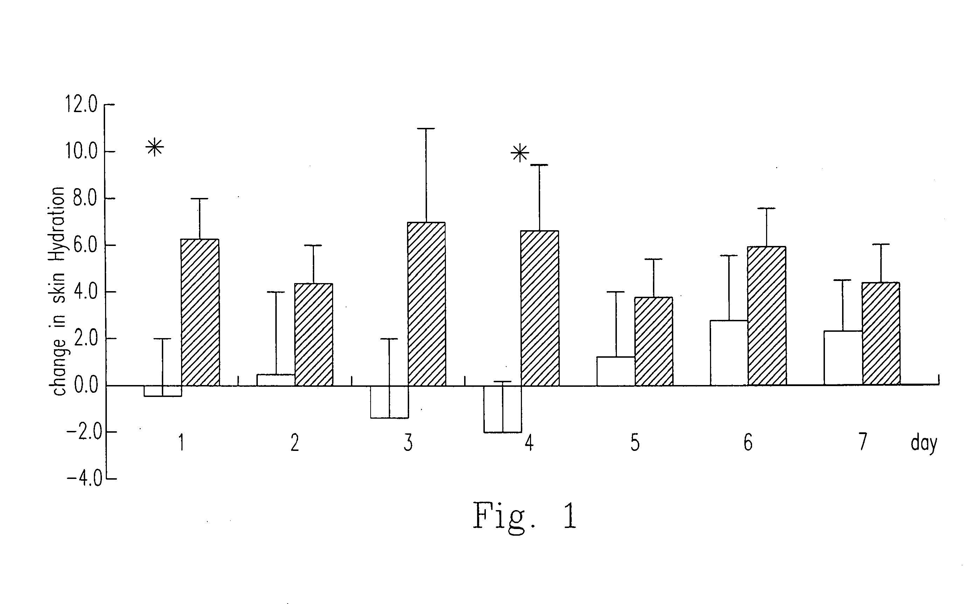

Because the experiments are proceeded on the left or right forehead skin of a same person respectively, the interference of the temperature, the moisture, the degrees of movements and sweat from the examinee can be eliminated. The amount of grease on the forehead in the test group is significantly lower than that of the control group by the statistical method of ANOVA (Analysis of variance). As shown in Figure 1, there is a tendency of becoming significance for the average difference in each week, and the significant difference of the change of the sebum amount appears in the first week after the usage of the statistical method of ANOVA, as shown in Table 2 (pr>F 0.0001). But according to the time course, as shown in Table 3, the change of the sebum amount does not depend on time, i.e. the composition is administrated for a longer time and the efficacy will not increase with time (pr>F 0.2854). The above results show that the Lo-108 can effectively eliminate or depress the excretion of grease up to 12 hours, and it can temporarily decelerate the open amount of sebum (the major cause of acnes) from the sebaceous gland. This efficacy can be obtained in a short time. The efficacy of inhibiting sebum can be maintained for at least 4 weeks if the Lo-108 is administrated day by day.

**Table 2. The mount of grease on the skin of left and right foreheads (µg/cm²)**

| Time (weeks) | Lo-108 | Control |
|---|---|---|
| 0 week | 88.15±13.02 | 93.85±12.82 |
| 1 week | 82.80±12.40 | 94.00±13.52 |
| 2 week | 88.00±9.53 | 112.55±12.88 |
| 3 week | 81.75±11.24 | 118.8±12.66 |
| 4 week | 61.15±8.36 | 99.95±10.38 |

**Table 3. The difference of the amount of sebum**

| Source | degree of freedom (DF) | Anova SS | Mean Square | F Value | Pr > F (probability > F) |
|---|---|---|---|---|---|
| No. of patient | 19 | 229013.20 | 12053.33 | 6.88 | 0.0001 |
| Number of times of treat | 4 | 11278.55 | 2819.64 | 1.61 | 0.1740 |
| Treat | 1 | 27518.58 | 27518.58 | 15.71 | 0.0001 |
| Treat *time | 4 | 8869.17 | 2217.29 | 1.27 | 0.2854 |

| | | | | | |
|---|---|---|---|---|---|
| Note: treat *time means the interaction between treat and time. | | | | | |

Experiment 3: burning and recovering of a scar in an animal model

The age of male rats used in this experiment is 8 weeks, and the species is Wistar. These animals are fed in the experimental animal center in National Cheng Kung University, which is the only qualified center according to the animal culturing standard (SPF) in south Taiwan. The rats should be fed in a laboratory with an air conditioner, which the temperature is set as 25±1°C, and all the animals can eat and drink freely.

Burning Experiment

The experiment procedure is as the following steps, and the control is proceeded on each animal itself in this experiment. Each rat is anesthetized by pentobarbital at the dosage of 65mg/kg. After the rats are comatose, the back of each rat is divided into four part with the area of 4 cm², and then hairs on each part of the back is shaved using a razor. Then, a red-bum iron sheet (about 80-85°C) is put onto the four parts of the back for about 10 seconds, which will cause scald on the back of each rat. After these processes have been finished, the iron sheet is removed and the wound is simply sterilized and cleared with the hydrogen peroxide solution (37%). Then, the four parts are treated separately: the control part is not administrated with any drug, and the other three parts are administrated with vitamin E, the base of the composition and the test composition respectively. The dosage is provided to cover all the wound areas. After each part is treated, the wound is bandaged tightly to prevent bacteria infection. The dressings are changed and the wounds are observed daily at a regular interval. The wounds are reserved by taking pictures therefor on the same time everyday. Seven days later, the rats are sacrificed, and the tissues of the four parts are taken for pathological sections.

According to the recovery of burning wounds, the inflammation has little change at the part which is treated with the composition. The results show that there is little difference between the control part and the part treated with the base of the composition. There is no inflammation change at the part treated with the composition.

Experiment of the recovery of wounds

The age of male rats used in this experiment is also 8 weeks, and the species is Wistar. According to the procedures above-described, the back of each anesthetized rat is divided into four parts, each area is about 4cm², and then hairs of each part is cut by a razor. Then, a incision is made on each part of the back using a scalpel, wherein the length of wound is about 1 cm, and the wounds are deep enough to expose the muscle layer. Then, the wounds are simply sterilized and cleared with the hydrogen peroxide solution (37%). Similarly, four parts are treated respectively. The control part is administrated without any drugs, the other three parts are administrated with vitamin E, the base of the composition and the test composition respectively. The dosage is provided enough to cover all the wound areas. After each part is treated, the wound is bandaged tightly to prevent bacteria infection. The change of dressings should be done and the wound should be observed daily at a regular interval. The wounds are reserved by taking photos on the same time of each day. Finally, the efficacy difference among groups is compared according to the days of recovery. The fewer days it needs, the faster the rats recover.

According to the recovery of the wounds, it is found that the time for recovery is shorter if the wound is treated with the composition, which is about 7.13+1.27 days (N=8). Meanwhile in the control group, it takes about 11.00+2.24 days (N=8), and in the group treated with the base of composition, it takes about 10.13+1.62 days (N=8). There is no significantly difference (P>0.05) between the control group and the group treated with the base of composition.

Experiment 4: bacteriostasis

Three bacteria stains are used in the experiment: *Staphylococcus aureus* Methicillin Restant (ATCC33591), *Staphylococcus aureus* and *Propionibacterium acnes* (ATCC6919). All of them are cultured respectively with proper media, and used to analyse the Lo-110 with the concentration of 0.03 µl/ml, 0.1 µl/ml, 0.3 µl/ml, 1 µl/ml, 3 µl/ml, 10 µl/ml, 30 µl/ml and 100 µl/ml. The results show that the inhibitory concentration of Lo-110 is 100µl/ml.

Experiment 5: the moisturizing effect of the skin

24 examinees are selected, and they are not allergic to drugs for skins. All the examinees do not suffer skin diseases in the past three months, and there are no scars on their cheeks. Their ages are from 20-40 year-old, and half of them are male. The examinees are divided into 2 groups randomly. Before and during the experiment, the examinees can not use any materials on the test site (their cheeks), and they have to reduce sweat on cheeks and avoid being exposed to the sun.

There are 12 examinees in each group. The examinees of the first group use Lo-107, and those of the second group use Lo-108. Each of the examinees are applied with 1 ml of the formulation on the left cheek as an experimental group, and applied with nothing on the right cheek as a control.

The experiment starts at 4 p.m., and the examinees wash their faces with the neutral cleaner. After 15 minutes, the skin hydration baseline is measured, and the examinees are applied with the test composition. 30 minutes later, the residual composition is slightly removed by wet tissues, and the examinees wash their face with the neutral cleaner again. The skin hydration is measured 15 minutes later. It is measured on the cheeks with the instrument Comeometer CM 825 (Courage and Khazaka Ltd., Germany), and the results are analysed via the ANOVA method.

It is found that the moisturizing effect is not outstanding when both Lo-107 and Lo-108 are used on the skin of the forehead. If the formulations are used with cheeks, however, the moisture of the skin is significantly increased.

While the invention has been described in terms of what is presently considered to be the most practical and preferred embodiments, it is to be understood that the invention needs not be limited to the disclosed embodiment. On the contrary, it is intended to cover various modifications and similar arrangements included within the spirit and scope of the appended claims which are to be accorded with the broadest interpretation so as to encompass all such modifications and similar structures.

## Claims

1. A composition for use with a skin, comprising a vitamin C in an amount from 1% to 45% by weight, a vitamin B complex in an amount from 1% to 5% by weight, a carotene in an amount from 0.1% to 3% by weight, a vitamin E in an amount from 2% to 90% by weight, a flavor in an amount from 0.1% to 2% by weight, a thickening agent in an amount from 1% to 5% by weight, a surfactant in an amount from about 1% to 8% by weight and distilled water.

2. The composition according to claim 1, preferably comprising said vitamin C in an amount from 4% to 15% by weight, said vitamin B complex in an amount from 1% to 3% by weight, said carotene in an amount from 0.1% to 2% by weight and said vitamin E in an amount from 15% to 65% by weight.

3. The composition according to claim 1, wherein said skin is a human skin.

4. The composition according to claim 3, wherein said skin is a face skin.

5. The composition according to claim 3, wherein said skin is a limb skin.

6. A physiologically active composition comprising said composition according to claim 1.

7. The physiologically active composition according to claim 6 comprising a curative effect to one selected from a group consisting of acnes, comedos and zits.

8. The physiologically active composition according to claim 6 comprising a skin-caring effect.

9. The physiologically active composition according to claim 6 being used for keeping the moisture of a skin.

10. The physiologically active composition according to claim 6 being an antioxidant.
